# EUROPEAN PATENT SPECIFICATION

(11) **EP 3 629 708 B1**
(45) Date of publication and mention of the grant of the patent: **02.08.2023**
(21) Application number: 18728882.4
(22) Date of filing: 01.06.2018
(51) Int. Cl.: A01H 4/00, C12N 15/82

(54) **METHOD FOR AUTOMATED TRANSFORMATION OF A PLANT CELL PACK**
VERFAHREN ZUR AUTOMATISIERTEN UMWANDLUNG EINES PFLANZENZELLPAKETS
PROCÉDÉ DE TRANSFORMATION AUTOMATIQUE D'UN BLOC DE CELLULES VÉGÉTALES

(30) Priority: 02.06.2017 EP 17174319
(43) Date of publication of application: 08.04.2020
(73) Proprietor: Fraunhofer-Gesellschaft zur Förderung der angewandten Forschung e.V., 80686 München (DE)
(72) Inventor: GENGENBACH, Benjamin, 52070 Aachen (DE); BUYEL, Johannes, 41812 Erkelenz (DE)
(74) Representative: Altmann Stößel Dick Patentanwälte PartG mbB
(86) International application number: PCT/EP2018/064477
(87) International publication number: WO 2018/220181

(56) References cited:
- EP-A2- 1 366 822
- WO-A1-2013/113504
- WO-A1-2016/181171
- WO-A2-2008/028115
- JANET REED SCIOLI ET AL: "Cloning and characterization of a cDNA encoding the chloroplastic copper/zinc-superoxide dismutase from pea", PROC. NATL. ACAD. SCI. USA,, vol. 85, 1 October 1988 (1988-10-01), pages 7661-7665, XP001313064,
- ZHU Q ET AL: "ACCURATE IN VITRO TRANSCRIPTION FROM CIRCULARIZED PLASMID TEMPLATES BY PLANT WHOLE CELL EXTRACTS", THE PLANT JOURNAL, BLACKWELL SCIENTIFIC PUBLICATIONS, OXFORD, GB, vol. 7, no. 6, 1 June 1995 (1995-06-01), pages 1021-1030, XP002072845, ISSN: 0960-7412, DOI: 10.1046/J.1365-313X.1995.07061021.X
- GUANGBO LIU ET AL: "High-throughput transformation of Saccharomyces cerevisiae using liquid handling robots", PLOS ONE, vol. 12, no. 3, 20 March 2017 (2017-03-20) , page e0174128, XP055416910, DOI: 10.1371/journal.pone.0174128
- DLUGOSZ ELIZABETH M ET AL: "Development of a Robotic Platform for Automated Protoplast Isolation, Transformation, and Screening of Plant Suspension Cultures", IN VITRO CELLULAR & DEVELOPMENTAL BIOLOGY. ANIMAL, SPRINGER US, NEW YORK, vol. 52, no. Suppl. 1, 31 May 2016 (2016-05-31), page S53, XP009500881, ISSN: 1071-2690

## Description

The present invention relates to a method for transformation of cultured plant cells comprising a) providing a plant cell package (PCP) of cultured plant cells to be transformed, b) contacting said PCP with a liquid comprising a transforming agent for an incubation period, c) removing said liquid comprising a transforming agent; and, thereby, d) transforming said cultured plant cells, wherein the liquid comprising the transforming agent is applied to the PCP in step b) by automated equipment, preferably at a rate of at most 50 µl/s. Moreover, the present invention relates to transformed plant cells, uses and methods related thereto.

Many efforts have been made to provide plant-based systems for recombinant production of secondary metabolites or proteins. Plant cell cultures, as well as intact plants can, in principle, be used for such purpose. To achieve the required changes in metabolism and to make plant cells produce a recombinant gene or product thereof, in principle two methods can be used, namely on the one hand, providing stable transgenic plants or plant cell cultures, or, on the other hand, providing transiently transfected plants or plant cell cultures. For transformation, usually, inert carrier particles (e.g. microparticles made from gold, "gene gun"), bacteria (in particular Agrobacterium tumefaciens strains), viruses (e.g. tobacco mosaic virus, TMV) or a combination of the latter two ("magnifection", WO 2002088369 A1) may be used.

In general, transient transformation is of advantage where stable transformation is not required and where results shall be obtained quickly, since selection of transgenic cells and their culture under selective pressure are not required. This applies even more so to intact plants.

In order to provide optimal constructs for transgenes, usually prescreening experiments are performed to test a large number of possible candidates and/or conditions on a small scale. At present, it is usual to perform prescreening by manually injecting transformation solutions into leaf material and to harvest and extract samples by hand. However, caused by differences in leaf position, leaf age, and also small genotypic or phenotypic differences between different plants, there may be strong variations within a group of replicas, which reduces the value of such experiments in terms of transferability and scalability. Moreover, with an increasing number of constructs to be tested, the workload, and thus time and costs, increases drastically.

Plant cell packages (PCPs) provide the possibility to perform prescreening experiments in plant cell culture (EP 2623603 A1), having the advantage of providing a homogeneous population of plant cells, which can be obtained e.g. in batch culture as taught by EP 3136841 A1. Moreover, in experiments with PCPs, environmental parameters such temperature, nutrition supply and the like can be more easily controlled than in intact plants.

Besides PCPs, also cell-free expression systems have been proposed, including systems containing micro-vesicles of the endoplasmic reticulum and, accordingly, providing for post-translational modification of proteins (cg. e.g. WO 2015165583 A1 and Buntru et al. (2014), BMC Biotechnol. 14:37.

Despite the aforesaid advance in prescreening systems, there is still a requirement for improved means and methods for performing such screenings due to the number of time consuming and costly hands-on steps. It is therefore an objective of the present invention to provide means and methods to comply with the aforementioned needs, avoiding at least in part the disadvantages of the prior art.

The aforementioned problem is solved by the methods and means described herein. Preferred embodiments, which might be realized in an isolated fashion or in any arbitrary combination are listed in the dependent claims and are described in this specification.

Accordingly, the present invention relates to a method for transformation of cultured plant cells comprising
a) providing a plant cell package (PCP) of cultured plant cells to be transformed,
b) contacting said PCP with a liquid comprising a transforming agent for an incubation period,
c) removing said liquid comprising a transforming agent; and, thereby,
d) transforming said cultured plant cells,
wherein the liquid comprising the transforming agent is applied to the PCP in step b) by automated equipment, preferably at a rate of at most 50 µl /s and wherein said providing a PCP in step a) and said removing said liquid comprising a transforming agent in step c) comprise centrifuging the PCP at of from 500 x g to 3500 × g

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Furthermore, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not otherwise indicated, the term "about" relates to the indicated value with the commonly accepted technical precision in the relevant field, preferably relates to the indicated value ± 20%, more preferably ± 10%, most preferably ± 5%.

The method of the present invention, preferably, is an in vitro method. Moreover, it may comprise steps in addition to those explicitly mentioned above. For example, further steps may relate, e.g., to growing plant cells under specific conditions for step a), or lysing the plant cells in the plant cell pack after step d). Moreover, one or more of said steps may be performed by automated equipment as specified herein below. Preferably, at least one, more preferably at least two, even more preferably all three of steps a) to c) are performed by automated equipment. Most preferably, all steps are performed by automated equipment, preferably including the steps of providing the liquid comprising a transforming agent.

Preferably, in the method of the present invention, media and equipment are sterilized, however, more preferably, no special precautions for sterility during sample handling are taken. Thus, preferably, the steps of the method may be performed under conditions ensuring sterility, e.g. under laminar flow devices; more preferably, however, it is preferred to use sterilized media and equipment as the only sterility precautions.

The term "plant cell" is known to the skilled person to relate to any cell of a member of the kingdom plantae within the domain eukaryota. Preferably, the plant cell is a cell from a plant which belongs to the superfamily Viridiplantae, preferably Tracheophyta, more preferably Spermatophytina, most preferably monocotyledonous and dicotyledonous plants including fodder or forage legumes, ornamental plants, food crops, trees or shrubs. Preferably, the plant cell is a cell from a plant selected from the list consisting of Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. (canola, oilseed rape, turnip rape), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Omithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, and Zizania palustris, Ziziphus spp. More preferably, the plant cell is a tobacco (Nicotiana tabacum) cell, a carrot (Daucus carota) cell, or a wheat (Tricium aestivum) cell. The plant cell may be a cell from or a cell derived from a whole plant, a plant part, a plant organ, or a plant tissue. Thus, the term includes, preferably, cells from and cells derived from seeds, shoots, stems, leaves, roots (including tubers), and flowers.

The term "cultured plant cell", as used herein, relates to a plant cell maintained as a single cell or as a cell aggregate in a culture medium, including protoplasts. Preferably, the cultured plant cell is a plant cell maintained in suspension culture. Methods and culture media for inducing callus formation and initiating suspension culture of plant cells are known in the art. A preferred medium for suspension culture of cells is Murashige & Skoog medium (Murashige & Skoog (1962), Physiologia Plantarum. 15 (3): 473). Preferably, at least one week, more preferably at least two weeks, before applying the method for transformation described herein, the cells are maintained in batch suspension culture, more preferably in continuous suspension culture. Preferred methods for culturing plant cells are described e.g. in WO 2015/165583 A1. In a preferred embodiment, plant cells are cultivated until single cell status is reached before transformation. Preferably, the cultured plant cells are grown to a wet mass density of from 25 g/l to 200 g/l, preferably of from 75 g/l to 100 g/l before transformation. The term "wet mass density" is understood by the skilled person; preferably, the term relates to the mass of cells per culture volume, determined by centrifuging off and weighing the mass of cells comprised in a defined volume of culture medium. Preferably, the cultured plant cells are concentrated to a wet mass density of at most 300 g/l, preferably at most 200 g/l before being converted into a plant cell pack, preferably by vacuum filtration or centrifugation, more preferably by sedimentation. Preferably, the cultured plant cells are maintained in a medium comprising of from 10 g/l to 50 g/l saccharose, preferably of from 15 g/l to 35 g/l saccharose, more preferably about 20 g/l saccharose, preferably for at least one week, more preferably at least two weeks, before applying the method for transformation described herein. Also preferably, the cultured plant cells are maintained in a medium comprising of from 1 mM to 10 mM phosphate ions, preferably of from 2 mM to 5 mM phosphate ions, more preferably about 2.7 mM phosphate ions, preferably for at least one week, more preferably at least two weeks, before applying the method for transformation described herein. Preferably, for dispensing cultured plant cells for generating a PCP, the medium comprising the plant cells is agitated, preferably by shaking or by stirring, more preferably by stirring, most preferably by stirring with a magnetic stirrer.

The term "plant cell package" (or "PCP") is known to the skilled person, e.g. from EP 2 623 603 A1, to relate a compacted package of plant cells, e.g. a pellet of plant cells. The PCP is provided by centrifuging cultured cells at of from 500g to 3500g, preferably of from 1500g to 2000g, wherein said centrifugation is performed for at least 15 s, preferably at least 30 s. More preferably, said centrifugation is performed for of from 15 s to 10 min, preferably of from 30 s to 1 min. Preferably, after centrifugation, the supernatant is removed. More preferably, the PCP is provided by centrifuging cultured plant cells onto a porous support; preferably, the porous support comprises or is a filter, more preferably, the porous support is a filter plate, most preferably a multiwell filter plate. Multiwell filter plates are, in principle, known to the skilled person and are commercially available in a variety of formats. Preferably, the porous support has pores with an average diameter of from 1 µm to 200 µm, preferably of from 30 µm to 40 µm. Preferably, the PCP is a compacted package of plant cells, more preferably having a mass density of from 0.1 g/cm³ to 0.9 g/cm³, most preferably of from 0.4 g/cm³ to 0.6 g/cm³. Preferably, the generation of the PCP is fully automated, i.e. preferably, the steps of pipetting an appropriate volume of cultured plant cells, preferably having a pre-adjusted wet mass density, into an appropriate container, preferably a multiwell filter plate, and centrifugation of the multiwell filter plate are performed by automated equipment, preferably without any interaction by the user. Preferably, the PCP is provided in a multiwell (multi-cluster) plate more preferably accommodating at most 0.5 ml of liquid per well, preferably at most 0.25 ml per well.

Preferably, for providing a plant cell package, an appropriate amount of cell mass is centrifuged onto a porous support, preferably a filter. The term "appropriate amount of cell mass" is understood by the skilled person and will essentially depend on the mass of cells required for subsequent steps and on the space provided by the container selected for maintenance of the PCP. Thus, e.g., for a single well of a 96-well plate, preferably a cell mass of from 50 mg to 100 mg, more preferably about 65 mg, may be used. Preferably, upon providing a PCP, a solidification means is included. The term "solidification means", as used herein, includes all means suitable to enhance overall stability of a PCP. Thus, the solidification means may be a flexible solidification means or a non-flexible solidification means. A flexible solidification means preferably is a means preventing the cell aggregates of the PCP from disengaging by becoming incorporated into the PCP structure; thus, preferably, the flexible solidification means is a fibrous compound, more preferably is a fibrous compound comprising or consisting of cellulose fibers. A non-flexible solidification means preferably is a means preventing the cell aggregates of the PCP from disengaging by providing a scaffold for PCP structure. Thus, preferably, the non-flexible solidification means is a rigid or semi-rigid compound providing external and/or internal support for the PCP; accordingly, the non-flexible solidification means may be added to a porous support before the cultured cells are applied, or may be part of the porous support.

For clarity, the term "plant cell culture" is used herein for a culture of plant cells in suspension culture typical for plant cells or being provided as protoplasts, whereas the term "plant cell package" is used for the condensed package of plant cells provided as described. As will be understood by the skilled person, both a plant cell culture and a PCP will contain cultured plant cells, the difference between plant cell culture and a PCP lying, preferably, in the volume of remaining free extracellular liquid (i.e., preferably, growth medium), which is low in a PCP, such that the cells and cell aggregates in a PCP are essentially fixed in their relative positions. As will be appreciated, contacting said PCP with a liquid comprising a transforming agent comprises applying a volume of said liquid so low as to not distort the structure of the PCP. Thus, preferably, the method of the present invention comprises maintaining the cultured plant cells as a PCP during the whole transformation process.

The term "transformation", as used herein, relates to an introduction of a, preferably heterologous, polynucleotide into a plant cell. The polynucleotide introduced may be stably integrated into the genome of the plant cell and/or a plastid and/or a mitochondrion thereof. Optionally, the polynucleotide may comprise a guide RNA triggering the specific action of a CRISPR/Cas protein complex. Preferably, the polynucleotide introduced is not stably integrated, thus, preferably, transformation is transient transformation. More preferably, transformation is transient nuclear transformation.

The term "polynucleotide" as used herein, refers to a linear or circular nucleic acid molecule. The polynucleotide of the present invention shall be provided, preferably, either as an isolated polynucleotide (i.e. isolated from its natural context), in genetically modified form, or comprised in a vector as specified herein below. The term encompasses DNA and RNA, preferably the polynucleotide is DNA; also, the term comprises single as well as double stranded polynucleotides. Moreover, comprised are also chemically modified polynucleotides including naturally occurring modified polynucleotides such as glycosylated or methylated polynucleotides or artificially modified derivatives such as biotinylated polynucleotides. Preferably, the polynucleotide is a heterologous polynucleotide, i.e. a polynucleotide comprising a nucleic acid sequence not naturally occurring in the plant cell transformed. More preferably, the polynucleotide encodes a gene of interest, e.g. an expressible sequence encoding a polypeptide of interest.

The polynucleotide of the present invention may be comprised in a vector. The term "vector", preferably, encompasses plasmid and viral vectors as well as artificial chromosomes, such as bacterial or yeast artificial chromosomes. Moreover, the term also relates to targeting constructs which allow for random or site- directed integration of the targeting construct into genomic, plastid, and/or mitochondrial DNA. Such target constructs, preferably, comprise DNA of sufficient length for either homologous or heterologous recombination. The vector encompassing the polynucleotide of the present invention, preferably, further comprises selectable markers for propagation and/or selection in a bacterial and/or a plant cell.

More preferably, in the vector of the invention the polynucleotide is operatively linked to expression control sequences allowing expression in a plant cell or isolated fractions thereof. Thus, preferably, the vector is an expression vector, more preferably a transient expression vector. Expression of a polynucleotide comprises transcription of the polynucleotide, preferably into a translatable mRNA. Regulatory elements ensuring expression in eukaryotic cells, preferably plant cells, are well known in the art. They, preferably, comprise regulatory sequences ensuring initiation of transcription and translation and, optionally, poly-A signals ensuring termination of translation and stabilization of the transcript. Additional regulatory elements may include transcriptional as well as translational enhancers. Moreover, inducible expression control sequences may be used in an expression vector encompassed by the present invention. Such inducible vectors may comprise tet (tetracycline) operator sequences or sequences inducible by heat shock or other environmental factors. Suitable expression control sequences are well known in the art. Preferred vectors are also described in WO 2002/088369 A1 and references cited therein.

The term "contacting", as used herein, relates to bringing two compounds into close proximity or admixture, so as to allow the compounds to interact.

As used herein, the term "transforming agent" relates to any compound or mixture of compounds suitable to cause transformation of a cell, preferably a plant cell. Transforming agents for plant cell transformation are, in principle, known in the art. The skilled person is also aware that suitability of a specific transforming agent may depend on the species, type, and state of a plant cell to be transformed, as well as on other factors. Thus, in case the cultured plant cell is a protoplast, the transforming agent, preferably, is an isolated polynucleotide or a vector as specified herein above, preferably in admixture with a chemical compound enhancing transformation, e.g. polyethyleneimine. Cultured plant cells, preferably, are transformed by means of a viral and/or bacterial vector, by a gene gun method, or the like. Preferably, the transforming agent is a plant virus, more preferably a recombinant plant virus infecting the plant cell to be transformed, more preferably is a recombinant member of one of the virus families Virgaviridae, Potyviridae and Geminiviridae, most preferably is Tobacco mosaic virus, Tobacco etch virus, or Maize streak virus. More preferably, the transforming agent is a, preferably recombinant, bacterium, preferably from the family Rhizobiaceae, more preferably from the genus Agrobacterium, even more preferably is Agrobacterium tumefaciens, Agrobacterium radiobacter, or Rhizobium radiobacter, most preferably is Agrobacterium tumefaciens. Preferably, the Agrobacterium tumefaciens is Agrobacterium tumefaciens strain GV3101, more preferably Agrobacterium tumefaciens strain GV3101 comprising plasmid pMP90RK (Agrobacterium tumefaciens strain GV3101:pMP90RK). As will be understood by the skilled person, methods which are well known to those skilled in the art can be used to construct recombinant transforming agents; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

The term "liquid comprising a transforming agent", as used herein, relates to any liquid compatible with application to a PCP and with the transforming agent, comprising the transforming agent as specified herein below. Preferably, the liquid is an aqueous liquid, more preferably is water, even more preferably is a buffer. In case the transforming agent is a bacterium, e.g. an Agrobacterium, preferably, the liquid is an aqueous buffer, more preferably is a buffer comprising saccharose, glucose, essential minerals, and/or acetosyringone, most preferably is a buffer comprising saccharose at a concentration of about 50 g/l, glucose at a concentration of about 2 g/l, standard plant fertilizer (e.g. Ferty 2 MEGA (Planta Düngemittel GmbH)) at a concentration of about 0.5 g/l, and acetosyringone at a concentration of about 200 µM and having a pH of about 5.6. Preferably, the liquid comprising the transforming agent is a solution as specified above comprising recombinant bacteria, preferably Agrobacterium bacteria, at an optical density measured at 600 nm (OD₆₀₀) of from 0.1 to 1.5, preferably of from 0.2 to 1.0, more preferably of from 0.3 to 0.7, most preferably of about 0.5.

In a preferred embodiment, the method for transformation of cultured plant cells comprises a step or several steps of automated generation of a liquid comprising a transforming agent. Thus, preferably, the step of adjusting density of transforming agent, in particular adjusting density of transforming bacteria, is performed by automated equipment. More preferably, in addition, the preceding steps of harvesting transforming bacteria, resuspending the same, and determining density are also performed by automated equipment. Preferably, in such case, bacteria are cultured in a multiwell (multicluster) plate having the same number of wells as the plate used for generating PCPs. Also, preferably, said bacteria are pelleted after growth by centrifuging at of from 1000 x g to 3000 x g, preferably of from 1300 x g to 2300 x g, more preferably at about 1800 x g. In a preferred embodiment, said bacteria are pelleted after growth by centrifuging at of from 500 x g to 4000 x g, preferably of from 1300 x g to 3000 x g, more preferably at about 2400 x g. Also, preferably, said bacteria are resuspended by adding an appropriate amount of buffer and rotating the multiwell (multicluster) plate for at least 5 min. Also, preferably, the density of said resuspended bacteria, preferably, is determined by an automated plate reader and potentially required dilutions are automatically calculated and applied. Even more preferably, the further preceding step of cultivating said transforming bacteria is also performed by automated equipment. Thus, in case the transforming agent is a bacterium, most preferably, the liquid comprising the transforming agent is provided by an automated method, preferably a fully automated method comprising all steps starting from culturing said bacteria. Preferred conditions for preparing a solution comprising Agrobacterium bacteria as a transforming agent in a 96-well format are described herein in the Examples.

According to the present invention, the liquid comprising the transforming agent is applied to the PCP in step b) by automated equipment. Preferably, the liquid comprising the transforming agent is applied to the PCP by an automatic dispenser, preferably having an adjustable dispensing rate. Preferably, the rate of applying the liquid comprising the transforming agent onto the PCP is at most 50 µl/s, preferably if a single-opening dispenser is used. More preferably, the aforesaid dispensing rate is of from 1 µl/s to 50 µl/s, even more preferably is of from 10 µl/s to 50 µl/s. Preferably, the automated equipment used for dispensing according to the present invention is equipped with sterile pipet tips. Preferably, in the step of applying the liquid comprising the transforming agent to the PCP, the outlet of the automated equipment is brought to close proximity of the PCP. Preferably, "close proximity" in the aforesaid context means a distance of less than the diameter of an average drop of liquid comprising the transforming agent forming on the outlet of the automated equipment. Thus, preferably, in the step of applying the liquid comprising the transforming agent to the PCP, the distance between the surface of the PCP and the outlet of the dispenser is less than 5 mm, preferably less than 4 mm. More preferably, in the step of applying the liquid comprising the transforming agent to the PCP, the distance between the surface of the PCP and the outlet of the dispenser is of from 0.5 mm to 5 mm, even more preferably is of from 1 mm to 4 mm. Preferably, of from 0.5 ml to 2 ml of liquid comprising the transforming agent are applied per g of PCP, preferably wherein of from 1 ml to 2 ml of liquid comprising the transforming agent are applied per g of PCP, more preferably wherein about 1.5 ml of liquid comprising the transforming agent are applied per g of PCP. Preferably, the mass of PCP is estimated from the wet mass density of the culture and the volume thereof used.

The term "incubation period", as used herein, relates to a time between applying the liquid comprising the transforming agent and removing said liquid. Without wishing to be bound by theory, the incubation period is thought to be the time frame required for at least part of the transforming agent to contact the plant cells such that it will not be eluted upon removal of the liquid comprising the transforming agent. Thus, the incubation period may be short and, preferably is of from 10 min to 3 h, preferably of from 30 min to 60 min, in a preferred embodiment of from 20 min to 60 min.

The term "removing a liquid comprising a transforming agent" is understood by the skilled person. Preferably, said removing consists of removing the liquid comprising the transforming agent, without undertaking any measures to remove the transforming agent. Thus, removing said liquid comprising a transforming agent comprises centrifuging the PCP at of from 500 x g to 3500 x g, preferably of from 1500 x g to 2000 x g, preferably on a porous support, more preferably on the same porous support used for generating the PCP. Preferably, said centrifugation is performed for at least 15s, preferably at least 30s; more preferably, said centrifugation is performed for of from 15s to 10 min, preferably of from 30s to 1 min.

According to the method of transformation the present invention, the PCP is, after removal of the solution comprising a transforming agent, preferably, incubated. As, preferably, also the preceding steps, said incubation is preferably performed at the optimal growth temperature of the cultured plant cells. Thus, the temperature, preferably is of from 20°C to 35 °C, more preferably is of from 25°C to 30°C, most preferably is about 26 °C. Preferably, the atmosphere the PCP is incubated in is ambient air, preferably, with a relative humidity of from 50% to 100%, preferably at least 50%, more preferably at least 70%, even more preferably at least 80%, most preferably at least 90%. As will be understood by the skilled person, the incubation is performed to allow for transformation to occur and, optionally, for gene expression. Accordingly, incubation of PCP is for at least 12 h, preferably at least one day, more preferably at least two days, even more preferably at least five days, in a most preferred embodiment at least three days; or incubation is for of from 12 h to 3 weeks, preferably of from 3 days to 2 weeks, more preferably of from 4 days to 8 days, most preferably 5 or 6 days. In a preferred embodiment, incubation of PCP is for of from 12 h to 3 weeks, preferably of from 2 days to 2 weeks, more preferably of from 3 days to 8 days, most preferably 4 or 5 days. Preferably, PCPs are incubated in a filter plate as specified above, more preferably upside down, i.e. with the openings facing towards the floor. Most preferably, PCPs are incubated in filter plates in an inverted manner with the openings pointing to a water reservoir.

Optionally, the method for transforming a cultured plant cell comprises the additional steps of lysing said cultured plant cells, preferably lysing said plant cells by means of a lysis buffer. Preferably, the lysis buffer comprises a detergent, preferably an anionic detergent, most preferably sodium dodecylsulfate, and/or an agent chelating divalent magnesium cations (Mg²⁺). More preferably, the lysis buffer comprises sodium dodecylsulfate at a concentration of from 0.1% (w/v) to 10% (w/v), preferably 1% (w/v), and ethylenediaminetetraacetic acid (EDTA) at a concentration of from 1 mM to 200 mM, preferably about 50 mM. In a preferred embodiment, the lysis buffer comprises tris(hydroxymethyl)aminomethane (Tris) at a concentration of from 10 mM to 200 mM, preferably about 100 mM at a pH value of from 8.0 to 10.0, preferably about 9.0. Typically about 1 to 6 ml of said lysis buffer are brought in contact per gram of PCP biomass, preferably 2 to 4 ml of said lysis buffer are added per gram of PCP biomass. Preferably the addition of said lysis buffer takes place in the same porous support used for the generation of the PCP. More preferably the addition is performed in an automated manner. Preferably the action of the lysis buffer is enhanced by incubating the PCP together with the lysis buffer at elevated temperatures of from 22 to 100°C, more preferably of from 10 to 60 min, preferably on a shaker with a speed of from 200 to 2000 rpm, more preferably of 1000 rpm. More preferably, a mineral oil is added in order to prevent evaporation of water at elevated temperature; the volume of mineral oil, preferably is of from 50 to 200 µL, more preferably of from 60 to 80 µL. After lysis of the plant cells constituting the PCP, cell debris is removed, preferably, by means of centrifugation of from 200 x g to 2000 x g, more preferably about 1000 x g, preferably using the same porous support used during the generation of the PCP. In a preferred embodiment, cell debris is removed by means of centrifugation of from 500 x g to 4000 x g, more preferably about 1000 x g, preferably using the same porous support used during the generation of the PCP. Preferably, in such case, the debris-free cell lysate is collected in a multiwall plate containing the same number of cavities as the multiwall plate containing the porous support, in which case the plate used for collecting the debris-free cell lysate has been placed beneath the plate with the porous support prior to centrifugation. Preferably one, more preferably all steps are performed using automated equipment.

The term "automated equipment" is understood by the skilled person. Preferably, the term relates to any device or combination of devices performing the step or steps indicated in a manner which, once established, does not require interaction with a human operator. Thus, once established, a method can, in principle, be performed by automated equipment for any number of times without requiring controlling interaction. Suitable automated equipment is known to the skilled person and is commercially available, e.g. the equipment described herein in the Examples.

Advantageously, and surprisingly, it was found in the work underlying the present invention that applying the transformation solution to a PCP by automated equipment makes more consistent transformation rates possible. As shown in the Examples herein below, well-to-well variation in the method according to the present invention is very small. Since the method can be performed in miniaturized (e.g. 96-well) format, it is highly suitable for high-throughput applications. Preferably, and also advantageously, it was further found that removing liquids from the PCP by centrifugation provides for higher overall transformation and for less variation in transformation efficiency compared to removing liquids by vacuum. Furthermore removing liquids from the PCP by centrifugation was found to allow for higher sucrose concentrations in the liquid comprising a transforming agent.

The definitions made above apply mutatis mutandis to the following. Additional definitions and explanations made further below also apply for all embodiments described in this specification mutatis mutandis.

Also described but not claimed is a transformed plant cell obtained or obtainable by the method according to the method for transformation of cultured plant cells according to the present invention.

As used herein, the term "transformed plant cell" relates to a plant cell comprising at least one heterologous polynucleotide. Thus, a transformed plant cell obtained or obtainable by the method according to the method for transformation of cultured plant cells according to the present invention is a plant cell comprising at least one heterologous polynucleotide, said heterologous polynucleotide having been introduced into said plant cell by the aforesaid method of the present invention. Since the transformation may be a stable transformation, and since a plant may be regenerated from the aforesaid transformed cell, the present invention also relates to a plant or plant part comprising at least one of the aforesaid transformed plant cells.

Also described but not claimed is the use of a transformed plant cell obtained or obtainable according to the method according to the present invention for high-throughput screening.

The present invention further relates to a method for providing a lysate of a transformed plant cell comprising
a) providing a PCP obtained according to the method for transformation of cultured plant cells according to the present invention, and the further steps of
b) incubating the PCP under conditions suitable for transformation to occur,
c) lysing the plant cells comprised in the PCP, and, thereby
d) providing a lysate of a transformed plant cell.

The method for providing a lysate of the present invention, preferably, is an in vitro method.

Moreover, it may comprise steps in addition to those explicitly mentioned above. For example, further steps may relate, e.g., to culturing plant cells before step a), or extracting and/or analyzing cellular constituents after step d). Moreover, one or more, preferably all, of said steps may be performed by automated equipment.

The term "lysate", as used herein, relates to any extract comprising cellular constituents of a transformed plant cell and, preferably, containing no or only a low number of intact plant cells. Preferably, the number of intact plant cells is less than 1000/ml, more preferably less than 100/ml. As will be understood, the transformation efficiency of the transformation method of the present invention will typically be below 100%, but, preferably, be above 80%, so the lysate will be a mixture of cell constituents of transformed and untransformed cells. Preferred methods for incubating the PCP and for lysing plant cells have been discussed herein above.

Furthermore, the present invention relates to a plant cell lysate obtainable by the method for providing a lysate of the present invention.

The present invention also relates to a method for causing transcription and, optionally, translation of at least one nucleic acid sequence in vitro in a cell-free system, comprising
a) providing a cell-free extract comprising cellular constituents required for transcription and/or translation of a polynucleotide
b) contacting the cell-free extract of step a) with a polynucleotide comprising an expressible nucleic acid sequence, and, thereby
c) causing transcription and, optionally, translation of said nucleic acid sequence.

The method for causing transcription of the present invention, preferably, is an in vitro method. Moreover, it may comprise steps in addition to those explicitly mentioned above. For example, further steps may relate, e.g., to culturing plant cells before step a), or extracting and/or analyzing gene expression products after step c). Also, the method may comprise adjusting the concentration of the polynucleotide comprising an expressible nucleic acid sequence to a predetermined target value before step b). Moreover, one or more, preferably all, of said steps may be performed by automated equipment.

The term "cell-free extract", as used herein, relates to an extract comprising in a functional form all components required for transcription and optionally translation to occur. As used herein, the term "cell-free" is not used in an absolute, but a relative sense. Preferably, an extract containing no or only a low number of intact plant cells is considered cell-free. Thus, preferably, an extract is cell-free if the number of intact plant cells is less than 1000/ml, more preferably less than 100/ml. Means for providing suitable cell-free extracts have been described e.g. in WO 2015/165583 A1.

Preferably, in the method for causing transcription, the cell-free extract is dispensed into wells of a multi-cluster plate in a fully automatized fashion by automated equipment. Also preferably, some, more preferably all, further pipetting steps are fully automatized. Preferably, determining the concentration of the polynucleotide comprising an expressible nucleic acid sequence and adjusting the concentration to a target value are also performed by automated equipment, in particular in case the method is performed in a multiwell (multicluster) plate format. Preferably, the method further comprises contacting said cell-free extract with a heterologous RNA polymerase, preferably T7 RNA polymerase; and/or comprises contacting said cell-free extract with a polynucleotide comprising an expressible gene encoding a heterologous RNA polymerase, preferably T7 RNA polymerase. Also preferably, the cell-free extract was obtained from plant cells obtained according to the method for transforming plant cells according to the present invention or wherein said cell-free extract is a lysate of a transgenic plant cell according to the present invention.

### Figure Legends

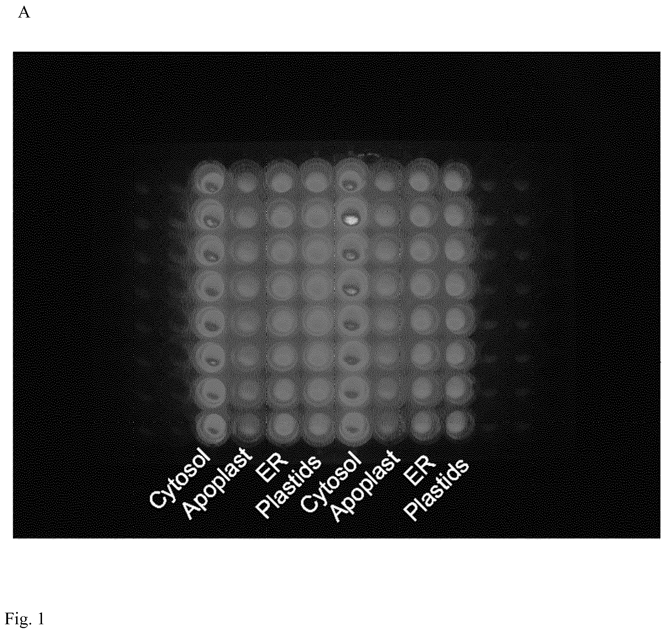

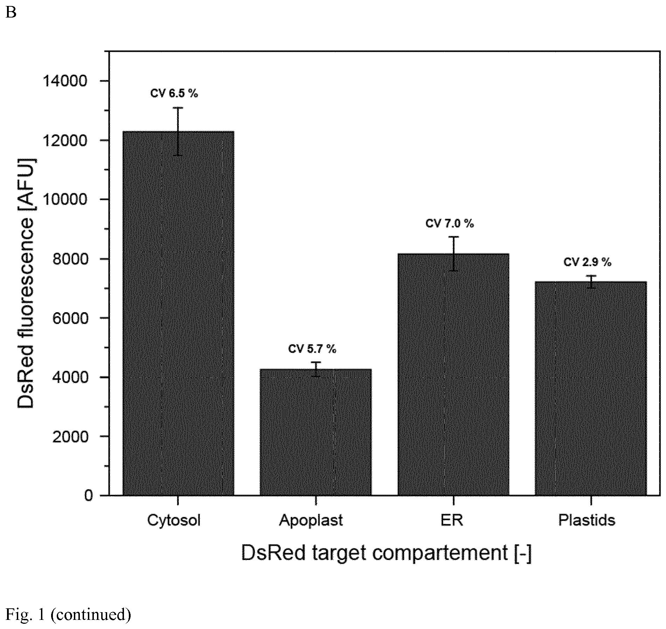

Fig. 1: A) Fluorescence of plant cell extracts obtained as described in the Examples using 50 g L⁻¹ sucrose in infiltration buffer from cells transformed with plasmid encoding DsRed-Fusion constructs targeting the indicated cell compartments. B) Quantification of fluorescence of the extracts shown in A); fluorescence was measured for each well and the values measured for corresponding wells were averaged; coefficient of variation (CV) is indicated; y-axis: average DsRed fluorescence in arbitrary fluorescence units (AFU).

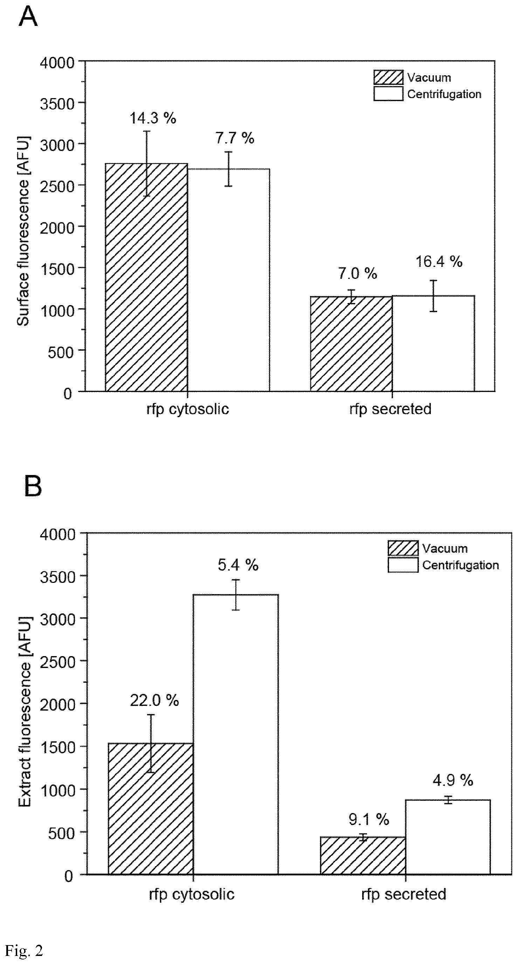

Fig. 2: Quantification of fluorescence of cells and extracts prepared according to Example 7 using 50 g L⁻¹ sucrose in infiltration buffer. A) fluorescence on average surface fluorescence; B) average extract fluorescence; coefficient of variation (CV) is indicated; y-axis: average DsRed fluorescence in arbitrary fluorescence units (AFU).

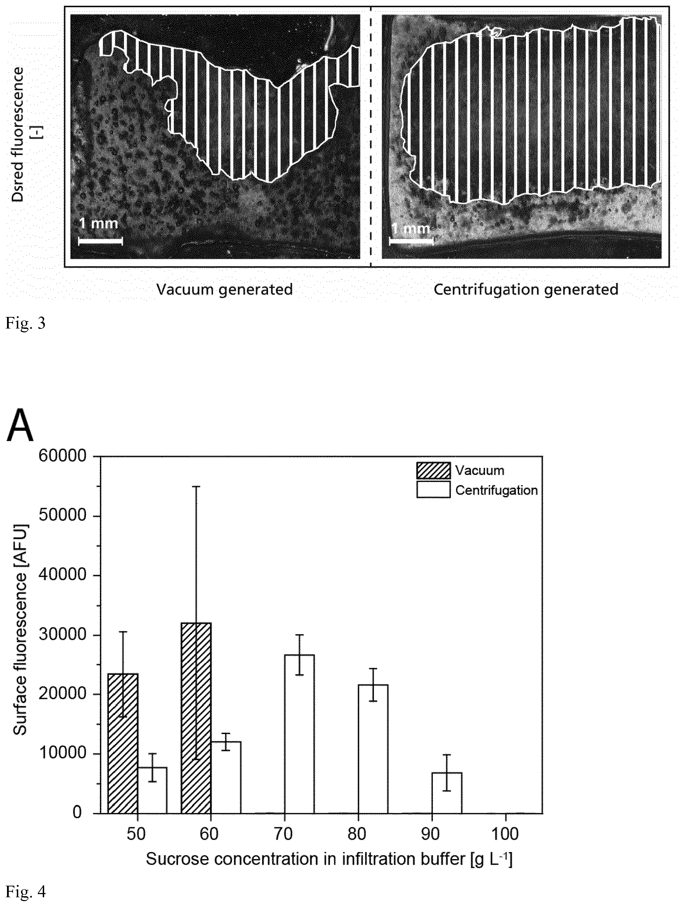

Fig. 3: Comparison of DsRed fluorescence pattern in Nicotiana tabacum BY-2 PCPs generated by vacuum or centrifugation according to Example 7 using 50 g L-1 sucrose in infiltration buffer. DsRed in PCP sections (hatched area) was visualized by fluorescence microscopy (530Ex/590Em).

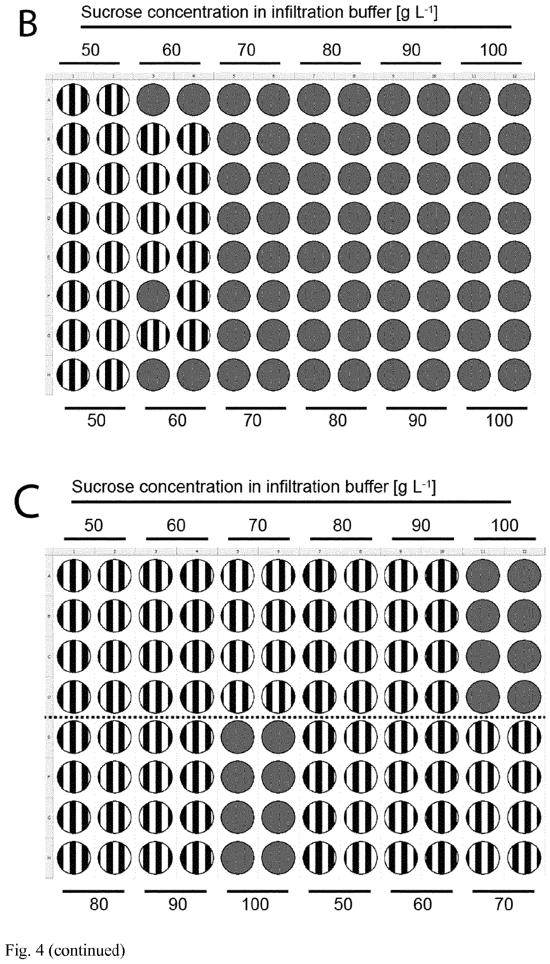

Fig. 4: Influence of sucrose concentration in infiltration buffer on DsRed expression in vacuum- and centrifugation generated PCPs according to Example 7. A. Surface fluorescence of PCPs four days after infiltration (559Ex/585Em, x̅ ± SD, n=16). B. Map of DsRed expression in PCPs generated by vacuum four days after infiltration, hatched=expression, grey=no expression. C. Map of DsRed expression in PCPs generated by centrifugation four days after infiltration.

The following Examples shall merely illustrate the invention. They shall not be construed, whatsoever, to limit the scope of the invention.

### Example 1: Devices and control

All pipetting steps and plate movements were performed by the automated pipetting station JANUS G3 (PerkinElmer). Depending on the volume to be transferred, appropriate single-use tips and pipetting parameters were selected. Control and data exchange between peripheral devices was provided by JANUS WinPREP Software (Version 5.1). Volume calculations were performed by user-provided scripts.

### Example 2: Preparation of Agrobacterium tumefaciens for PCP Infiltration

Agrobacterium tumefaciens GV3101(pMP90RK) cells, carrying one of the plant expression vectors pTRAc-rfp-H, pTRAc-rfp-ERH, pTRAc-rfp-AH oder pTRAc-TPrfp-H, which are all based on pTRA (Sack et al. (2015), Plant Biotechnol. J. 13: 1094). were grown at 28°C in 500 µl per well PAM Medium (soy peptone 20 g/l; yeast extract 0.5 g/l; fructose 5 g/l; magnesium sulfate (MgSO₄) 1 g/l, pH 7.0; with Carbenicillin (50 mg/l) and Kanamycin (25 mg/l) added) in a 96-well "Deep-Well" multicluster plate at 1000 rpm on an integrated rotary shaker having an amplitude of 2 mm to an OD₆₀₀ of 6.0. Bacterial cells were pelleted at 1800g for 4 min, supernatant was discarded. The pellet was resuspended in 200 µl sterile infiltration buffer (sucrose 50 g/l; glucose monohydrate 2 g/l; 0.5 g/l Ferty 2 MEGA (Planta Düngemittel GmbH); 200 µM acetosyringone; pH 5.6) at 1000 rpm and 2 mm amplitude for 8 min. The OD₆₀₀ of a 1:20 dilution was determined in a 96-well plate by an integrated plate reader, and all bacterial suspensions in the respective wells were normalized to an OD₆₀₀ of 0.4 into a fresh 96-weel plate.

### Example 3: Generation of PCPs

Nicotiana tabacum BY-2 were grown in continuous culture in Murashige & Skoog Medium (sucrose 20 g/l; MS salts (minimal organics, Fa. Duchefa) 4,3 g/l; KH₂PO4 200 mg/l; myo-inositol 100 mg/l; HCl-thiamine 1 mg/l; 2,4-Dichlorophenoxyacetic acid 0.2 mg/l, pH 5.0) at 26°C until 80 g/l wet mass density was reached. 2 1 of culture were withdrawn, left to sediment for about 45 min and were concentrated to a wet mass density of 200 g/l ± 5% by decanting medium.

From a sterile reservoir and under constant stirring with a magnetic stirrer, 300 µl of concentrated culture were transferred per well to an Agroprep Advance PP/PE 30-40 µm Filter Plate (Fa. Pall) and covered by a lid. The plate was loaded into the integrated centrifuge; medium was removed by centrifugation at 1800 x g for 1 min and collected by underlaying the filter plate with a reservoir plate.

### Example 4: Agrobacterium infiltration

To the PCPs of Example 3, 90 µl each of the normalized Agrobacterium-suspension was added dropwise, followed by incubation for 60 min at 22°C. Thereafter, the plate was reloaded into the internal centrifuge and was centrifuged at 1800 x g for 1 min to remove infiltration solution.

### Example 5: Incubation of PCPs

The filter plate with infiltrated PCPs was inverted and incubated over a 1-well "Deep-Well" reservoir for 72 h at 26°C and 80% relative humidity. The reservoir was filled with 150 ml H₂O and had an open space of 160 cm³; the connection between the filter plate and the reservoir was sealed air-tight.

### Example 6: Measuring fluorescence of PCPs

After incubation, the filter plates containing the PCPs were transferred into the integrated plate reader and fluorescence of DsRed protein in the PCPs was directly measured (excitation: 559 nm; emission 585 nm). Thereafter, the filter plate was sealed by a Silicone/PTFE-mat having premanufactured septum openings (Webseal mat M115, Fa. Thermo Scientific) and 200 µl of extraction buffer (sodium dodecylsulfate 1 g/l; ethylenediaminetetraacetic acid (EDTA) 50 mM; pH 8.0) were added to each well.

For lysis, the filter plates were kept at 800 rpm with 2 mm amplitude for 30 min at 65°C on an integrated rotary shaker. After lysis, the filter plate was transferred into the integrated centrifuge and centrifuged at 1800 x g for 1 min, wherein the lysate was recovered in a 96-well plate underlaid to the filter plate. With the extracts recovered, fluorescence measurements were performed as described above; the results are shown in Fig. 1.

### Example 7: Comparison of medium removal methods

Continuously cultured BY-2 cells were concentrated to 200 g fresh biomass/l and 300 µl of the cell suspension were transferred in sterile Agroprep Advance PP/PE 30-40 µm filter plates (Pall GmbH, Dreieich, Germany). Excess medium was removed by either centrifugation at 1800 rcf for one minute or by applying a vacuum of 800 mbar on a chromabond vacuum manifold for ~10 seconds (Macherey-Nagel, Düren, Germany).

Agrobacterium tumefaciens cells, grown in PAM liquid media (Carbenicillin 50 µg/ml, Kanamycin 25 µg/ml), were resuspended and adjusted to an OD600nm of 0.4 in 1x infiltration buffer (0.5 g/l Fertilizer MEGA2, 50 - 100 g/l sucrose, 2 g/l glucose monohydrate, 200 µM acetosyringone) and 100 µl were dropped onto each PCP, incubated for 1h and then removed by centrifugation or vacuum as described above.

Plates were inverted over 150 ml H₂O on a one well deep well plate with sealed edges and incubated at 26°C and 80% relative humidity (RH) for three days.

Surface fluorescence of PCPs and subsequently fluorescence of extract generated by mechanical lysis in extraction buffer (40 mM disodium hydrogen phosphate, 10 mM sodium dihydrogen phosphate, 10 mM sodium metabisulfite; 3 mL per gram biomass) using a bead mill (MM 300, Retsch GmbH, Han, Germany) were measured at 559nm (Excitation) / 585nm (Emission) with three reads per center of well.

Sections of about 0.5-1 mm thickness were cut from PCPs. Localization of DsRed expression in PCP section was visualized by fluorescence microscopy at 530nm (Excitation) / 590 nm (Emission).

Results: Averaged surface fluorescence of PCPs (n=5) showed similar expression levels of cytosolic (-2700 arbitrary fluorescence units (AFU)) and secreted (-1100 AFU) red fluorescent protein (rfp) for PCPs generated by vacuum or centrifugation. However, coefficient of variance (CV) of PCPs generated by vacuum was ~2-fold higher in case of cytosolic rfp and ~2-fold lower in case of secreted rfp compared to vacuum generated PCPs (Fig. 2A).

Averaged extract fluorescence of PCPs generated by centrifugation was ~2-fold higher for both cytosolic (-3200 AFU) and secreted (-1000 AFU) rfp compared to the vacuum method. CV of PCPs generated by centrifugation was ~4-fold lower for cytosolic and ~2-fold lower for secreted rfp, respectively (5.4%, 4.9%, Fig. 2B).

Fluorescence at 590 nm in the PCP generated by vacuum was mainly detected on the surface of the PCP, whereas PCPs generated by vacuum showed fluorescence throughout the whole body of the PCP (Fig. 3).

This indicates higher and more homogenous recombinant protein expression throughout the whole PCP for the centrifugation based method, whereas PCPs generated by vacuum predominantly show comparably high expression levels only on the surface of the PCP.

PCPs generated by centrifugation started to collapse at sucrose concentrations of 60 g L-1 in the infiltration buffer (Fig 4A), resulting in large inter-sample variance (coefficient of variance >70%) (Fig. 4 B) and no detectable DsRed expression at concentrations >70 g L-1 sucrose after four days of incubation. PCPs generated by vacuum remained intact and homogeneously expressed recombinant DsRed protein up to sucrose concentrations of 90 g L-1 (Fig. 4 A, C).

## Claims

1. A method for transformation of cultured plant cells comprising
a) providing a plant cell package (PCP) of cultured plant cells to be transformed,
b) contacting said PCP with a liquid comprising a transforming agent for an incubation period,
c) removing said liquid comprising a transforming agent; and, thereby,
d) transforming said cultured plant cells,
wherein the liquid comprising the transforming agent is applied to the PCP in step b) by automated equipment, preferably at a rate of at most 50 µl/s,
wherein said PCP is a compacted package of cultured plant cells, and
wherein said providing a PCP in step a) and said removing said liquid comprising a transforming agent in step c) comprise centrifuging the PCP at of from 500 x g to 3500 x g.

2. The method of claim 1, wherein said providing a plant cell package comprises centrifuging an appropriate amount of cell mass onto a porous support, preferably a filter.

3. The method of claim 1 or 2, wherein said porous support is a multiwell filter plate, preferably having pores with an average diameter of from 30 µm to 40 µm.

4. The method of any one of claims 1 to 3, wherein said PCP is a compacted package of plant cells having a mass density of from 0.1 g/cm³ to 0.9 g/cm³, preferably of from 0.4 g/cm³ to 0.6 g/cm³.

5. The method of any one of claims 1 to 4, wherein said method comprises adding a solidification means to said cultured plant cells.

6. The method of any one of claims 1 to 5, wherein said cells are cultivated in a medium comprising of from 10 g/l to 50 g/l saccharose, preferably of from 15 g/l to 35 g/l saccharose, more preferably about 20 g/l saccharose before step a).

7. The method of any one of claims 1 to 6, wherein said cells are cultivated in a medium comprising of from 1 mM to 10 mM phosphate ions, preferably of from 2 mM to 5 mM phosphate ions, more preferably about 2.7 mM phosphate ions before step a).

8. The method of any one of claims 1 to 7, wherein said transforming agent is selected from the group consisting of isolated recombinant polynucleotides, recombinant plant viruses, and recombinant bacteria, preferably is recombinant bacteria, preferably, wherein said recombinant bacteria are bacteria of the family Rhizobiaceae, preferably Agrobacterium tumefaciens, Agrobacterium radiobacter or Rhizobium radiobacter), more preferably are bacteria of Agrobacterium tumefaciens strain GV3101, most preferably Agrobacterium tumefaciens strain GV3101: pMP90RK.

9. The method of any one of claims 1 to 8, wherein said liquid comprising the transforming agent is provided by an automated method, preferably a fully automated method comprising all steps starting from culturing said Agrobacterium bacteria, preferably, wherein said method is a fully automated method.

10. A method for providing a lysate of transformed plant cells comprising
a) providing a PCP obtained according to the method according to any one of claims 1 to 9, and the further steps of
b) incubating the PCP under conditions suitable for transformation to occur,
c) lysing the plant cells comprised in the PCP, and, thereby
d) providing a lysate of transformed plant cells.

11. A method for causing transcription and, optionally, translation of at least one nucleic acid sequence in vitro in a cell-free system, comprising
a) providing a cell-free extract comprising cellular constituents required for transcription and/or translation of a polynucleotide
b) contacting the cell-free extract of step a) with a polynucleotide comprising an expressible nucleic acid sequence, and, thereby
c) causing transcription and, optionally, translation of said nucleic acid sequence,
wherein said cell-free extract was obtained from plant cells obtained according to the method according to any one of claims 1 to 9.

12. The method of claim 11, wherein said method comprises contacting said cell-free extract with a heterologous RNA polymerase, preferably T7 RNA polymerase; and/or comprises contacting said cell-free extract with a polynucleotide comprising an expressible gene encoding a heterologous RNA polymerase, preferably T7 RNA polymerase.

13. The subject matter of any of the preceding claims, wherein said plant cells are cells selected from monocotyledonous or dicotyledonous plants, more preferably from tobacco (Nicotiana tabacum), carrot (Daucus carota) or wheat (Tricium aestivum).

## Patentansprüche

1. Verfahren zur Transformation von kultivierten Pflanzenzellen, das Folgendes umfasst:
a) das Bereitstellen eines Pflanzenzellpakets (PCP) von zu transformierenden kultivierten Pflanzenzellen,
b) das In-Kontakt-Bringen des PCP mit einer ein Transformationsmittel umfassenden Flüssigkeit für eine Inkubationszeit,
c) das Entfernen der ein Transformationsmittel umfassenden Flüssigkeit und dadurch
d) das Transformieren der kultivierten Pflanzenzellen,
wobei die das Transformationsmittel umfassende Flüssigkeit in Schritt b) mittels einer automatisierten Einrichtung vorzugsweise mit einer Rate von höchstens 50 µl/s auf das PCP einwirken gelassen wird,
wobei es sich beim PCP um eine verdichtete Packung von kultivierten Pflanzenzellen handelt und
wobei das Bereitstellen eines PCP in Schritt a) und das Entfernen der ein Transformationsmittel umfassenden Flüssigkeit in Schritt c) ein Zentrifugieren des PCP bei 500 x g bis 3500 x g umfassen.

2. Verfahren nach Anspruch 1, wobei das Bereitstellen eines Pflanzenzellpakets das Zentrifugieren einer zweckmäßigen Zellmassenmenge auf einem porösen Träger, vorzugsweise einem Filter, umfasst.

3. Verfahren nach Anspruch 1 oder 2, wobei der poröse Träger eine Filterplatte mit mehreren Wells ist, die vorzugsweise Poren mit einem mittleren Durchmesser von 30 µm bis 40 µm aufweist.

4. Verfahren nach einem der Ansprüche 1 bis 3, wobei das PCP eine verdichtete Packung von Pflanzenzellen mit einer Massendichte von 0,1 g/cm³ bis 0,9 g/cm³, vorzugsweise von 0,4 g/cm³ bis 0,6 g/cm³, ist.

5. Verfahren nach einem der Ansprüche 1-4, wobei das Verfahren das Hinzufügen eines Verfestigungsmittels zu den kultivierten Pflanzenzellen umfasst.

6. Verfahren nach einem der Ansprüche 1 bis 5, wobei die Zellen vor Schritt a) in einem Medium kultiviert werden, das 10 g/l bis 50 g/l Saccharose, vorzugsweise 15 g/l bis 35 g/l Saccharose, noch mehr bevorzugt etwa 20 g/l Saccharose umfasst.

7. Verfahren nach einem der Ansprüche 1 bis 6, wobei die Zellen vor Schritt a) in einem Medium kultiviert werden, das 1 mM bis 10 mM Phosphationen, vorzugsweise 2 mM bis 5 mM Phosphationen, noch mehr bevorzugt etwa 2,7 mM Phosphationen umfasst.

8. Verfahren nach einem der Ansprüche 1 bis 7, wobei das Transformationsmittel ausgewählt ist aus der Gruppe bestehend aus isolierten rekombinanten Polynucleotiden, rekombinanten Pflanzenviren und rekombinanten Bakterien, vorzugsweise rekombinante Bakterien ist, wobei die rekombinanten Bakterien vorzugsweise Bakterien der Familie Rhizobiaceae, vorzugsweise Agrobacterium tumefaciens, Agrobacterium radiobacter oder Rhizobium radiobacter, noch mehr bevorzugt Bakterien des Agrobacterium-tumefaciens-Stamms GV3101, am meisten bevorzugt des Agrobacterium-tumefaciens-Stamms GV3101:pMP90RK, sind.

9. Verfahren nach einem der Ansprüche 1 bis 8, wobei die das Transformationsmittel umfassende Flüssigkeit mittels eines automatisierten Verfahrens, vorzugsweise eines vollständig automatisierten Verfahrens, bereitgestellt wird, das alle Schritte beginnend von der Kultivierung der Agrobacterium-Bakterien umfasst, wobei es sich beim Verfahren vorzugsweise um ein vollständig automatisiertes Verfahren handelt.

10. Verfahren zur Bereitstellung eines Lysats von transformierten Pflanzenzellen, das Folgendes umfasst:
a) das Bereitstellen eines PCP, das gemäß einem Verfahren nach einem der Ansprüche 1 bis 9 erhalten wurde, und die weiteren Schritte des
b) Inkubierens des PCP unter Bedingungen, die für das Auftreten einer Transformation geeignet sind,
c) Lysierens der im PCP enthaltenen Pflanzenzellen und dadurch des
d) Bereitstellens eines Lysats von transformierten Pflanzenzellen.

11. Verfahren zum Bewirken einer Transkription und gegebenenfalls einer Translation mindestens einer Nucleinsäuresequenz in vitro in einem zellfreien System, das Folgendes umfasst:
a) das Bereitstellen eines zellfreien Extrakts, der Zellbestandteile umfasst, die für eine Transkription und/oder Translation eines Polynucleotids erforderlich sind
b) das In-Kontakt-Bringen des zellfreien Extrakts von Schritt a) mit einem Polynucleotid, das eine exprimierbare Nucleinsäuresequenz umfasst, und dadurch
c) das Bewirken einer Transkription und gegebenenfalls einer Translation der Nucleinsäuresequenz, wobei der zellfreie Extrakt aus Pflanzenzelle erhalten wurde, die gemäß dem Verfahren nach einem der Ansprüche 1 bis 9 erhalten wurden.

12. Verfahren nach Anspruch 11, wobei das Verfahren das In-Kontakt-Bringen des zellfreien Extrakts mit einer heterologen RNA-Polymerase, vorzugsweise T7-RNA-Polymerase, umfasst; und/oder das In-Kontakt-Bringen des zellfreien Extrakts mit einem Polynucleotid umfasst, das ein exprimierbares Gen umfasst, das für eine heterologe RNA-Polymerase, vorzugsweise T7-RNA-Polymerase, codiert.

13. Gegenstand nach einem der vorhergehenden Ansprüche, wobei es sich bei den Pflanzenzellen um Zellen handelt, die aus monokotylen und dikotylen Pflanzen, noch mehr bevorzugt aus Tabak (Nicotiana tabacum), Karotte (Daucus carota) oder Weizen (Tritium aestivum) ausgewählt sind.

## Revendications

1. Procédé de transformation de cellules végétales en culture comprenant
a) la fourniture d'un bloc de cellules végétales (PCP) de cellules végétales en culture à transformer,
b) la mise en contact dudit PCP avec un liquide comprenant un agent transformant pendant une période d'incubation,
c) l'élimination dudit liquide comprenant un agent transformant, et ainsi
d) la transformation desdites cellules végétales en culture,
dans lequel le liquide comprenant l'agent transformant est appliqué au PCP dans l'étape b) par un équipement automatisé, de préférence à un débit d'au plus 50 µl/s, dans lequel ledit PCP est un bloc compacté de cellules végétales en culture, et
dans lequel ladite fourniture d'un PCP dans l'étape a) et ladite élimination dudit liquide comprenant un agent transformant dans l'étape c) comprennent une centrifugation du PCP à 500 x g à 3 500 x g.

2. Procédé selon la revendication 1, dans lequel ladite fourniture d'un bloc de cellules végétales comprend la centrifugation d'une quantité appropriée d'une masse cellulaire sur un support poreux, de préférence un filtre.

3. Procédé selon la revendication 1 ou 2, dans lequel ledit support poreux est une plaque filtrante multipuits, ayant de préférence des pores dont le diamètre moyen est de 30 µm à 40 µm.

4. Procédé selon l'une quelconque des revendications 1 à 3, dans lequel ledit PCP est un bloc compacté de cellules végétales ayant une masse volumique de 0,1 g/cm³ à 0,9 g/cm³, de préférence de 0,4 g/cm³ à 0,6 g/cm³.

5. Procédé selon l'une quelconque des revendications 1 à 4, ledit procédé comprenant l'addition d'un moyen de solidification auxdites cellules végétales en culture.

6. Procédé selon l'une quelconque des revendications 1 à 5, dans lequel lesdites cellules sont cultivées dans un milieu comprenant de 10 g/l à 50 g/l de saccharose, de préférence de 15 g/l à 35 g/l de saccharose, plus particulièrement environ 20 g/l de saccharose avant l'étape a).

7. Procédé selon l'une quelconque des revendications 1 à 6, dans lequel lesdites cellules sont cultivées dans un milieu comprenant de 1 mM à 10 mM d'ions phosphate, de préférence de 2 mM à 5 mM d'ions phosphate, plus particulièrement environ 2,7 mM d'ions phosphate avant l'étape a).

8. Procédé selon l'une quelconque des revendications 1 à 7, dans lequel ledit agent transformant est choisi dans le groupe consistant en les polynucléotides recombinants isolés, les virus végétaux recombinants et les bactéries recombinantes, de préférence les bactéries recombinantes, de préférence dans lequel lesdites bactéries recombinantes sont des bactéries de la famille Rhizobiaceae, de préférence Agrobacterium tumefaciens, Agrobacterium radiobacter ou Rhizobium radiobacter), plus particulièrement les bactéries de la souche d'Agrobacterium tumefaciens GV3101, tout spécialement la souche d'Agrobacterium tumefaciens GV3101 : pMP90RK.

9. Procédé selon l'une quelconque des revendications 1 à 8, dans lequel ledit liquide comprenant l'agent transformant est fourni par une méthode automatisée, de préférence une méthode entièrement automatisée comprenant toutes les étapes à partir de la culture desdites bactéries Agrobacterium, ledit procédé étant de préférence un procédé entièrement automatisé.

10. Procédé de fourniture d'un lysat de cellules végétales transformées comprenant
a) la fourniture d'un PCP obtenu par le procédé selon l'une quelconque des revendications 1 à 9, et les étapes supplémentaires de :
b) incubation du PCP dans des conditions permettant à une transformation de se produire,
c) lyse des cellules végétales comprises dans le PCP et ainsi
d) fourniture d'un lysat de cellules végétales transformées.

11. Procédé destiné à provoquer une transcription et éventuellement une traduction d'au moins une séquence d'acides nucléiques *in vitro* dans un système acellulaire, comprenant :
a) la fourniture d'un extrait acellulaire comprenant les constituants cellulaires nécessaires à la transcription et/ou à la traduction d'un polynucléotide,
b) la mise en contact de l'extrait acellulaire de l'étape a) avec un polynucléotide comprenant une séquence d'acides nucléiques exprimable et ainsi
c) le fait de provoquer une transcription et éventuellement une traduction de ladite séquence d'acides nucléiques,
dans lequel ledit extrait acellulaire a été obtenu à partir de cellules végétales obtenues par le procédé selon l'une quelconque des revendications 1 à 9.

12. Procédé selon la revendication 11, ledit procédé comprenant la mise en contact dudit extrait acellulaire avec une ARN polymérase hétérologue, de préférence une ARN polymérase de T7, et/ou comprenant la mise en contact dudit extrait acellulaire avec un polynucléotide comprenant un gène exprimable codant pour une ARN polymérase hétérologue, de préférence une ARN polymérase de T7.

13. L'objet selon l'une quelconque des revendications précédentes, dans lequel lesdites cellules végétales sont des cellules choisies parmi les plantes monocotylédones ou dicotylédones, de préférence le tabac (Nicotiana tabacum), la carotte (Daucus carota) ou le blé (Tricium aestivum).
